# EUROPEAN PATENT APPLICATION

(11) **EP 4 817 594 A1**
(43) Date of publication of application: **07.10.2026**
(21) Application number: 24898172.2
(22) Date of filing: 28.11.2024
(51) Int. Cl.: A61K 8/64, A61K 8/9789, A61K 8/14, A61Q 19/00, A23L 33/18, A61K 38/06, A61K 9/127, A61P 29/00, A61P 17/00

(54) **SKIN CONDITION IMPROVEMENT COMPOSITION COMPRISING GLUTATHIONE-CONTAINING EXOSOME-LIKE VESICLES**

(30) Priority: 30.11.2023 KR 20230171817
(71) Applicant: Cosmax, Inc., Hwaseong-si, Gyeonggi-do 18622 (KR)
(72) Inventor: LEE, Eun Jung, Seongnam-si, Gyeonggi-do 13486 (KR); KANG, Yong Won, Seoul 01811 (KR); RYU, Yang Hwan, Seoul 01811 (KR); WANG, Hye Soo, Seoul 01811 (KR); RYU, Jeoung Jin, Seongnam-si, Gyeonggi-do 13486 (KR); KANG, Seung Hyun, Seongnam-si, Gyeonggi-do 13486 (KR)
(74) Representative: Croce, Valeria
(86) International application number: PCT/KR2024/019201
(87) International publication number: WO 2025/116578

(57) **Abstract**

The present disclosure relates to a composition for skin condition improvement, including glutathione-containing exosome-like vesicles. The glutathione-containing exosome-like vesicles, in which glutathione, which is a substance useful for skin, is loaded in exosome-like vesicles isolated from plant callus, have an excellent anti-inflammatory effect as compared to the application of glutathione alone. Thus, the composition according to an aspect can be effectively used as a composition for skin condition improvement.

## Description

### Technical Field

The present disclosure relates to a composition for skin condition improvement including glutathione-containing exosome-like vesicles.

### Background Art

Glutathione is a tripeptide including glutamate, cysteine, and glycine, and is known as a useful nutrient having strong antioxidant activity. Accordingly, glutathione is used as a raw material for health functional foods and pharmaceuticals, and even for cosmetics. However, in spite of such usefulness, glutathione has a problem in that it is difficult to absorb because it has very low permeability across cell membranes.

An exosome is an extracellular vesicle (EV) having a size of several tens to several hundreds of nanometers, which is composed of a phospholipid bilayer having the same structure as a cell membrane. Exosomes are intercellular signaling mediators secreted by cells, and are known to regulate the behavior of various cells, and research on components and functions of exosomes is actively being conducted. Meanwhile, plant callus is a mass of cells formed when plant tissue is wounded to regenerate the wounded site, and because callus cells have totipotency to differentiate into a whole plant through treatment with a plant growth regulator or the like, the callus is also referred to as plant stem cells.

Recently, studies have been conducted to use, as a cosmetic material, exosomes derived from plant callus (*see* KR 10-2023-0052827), but attempts to use exosomes as carriers capable of stably retaining glutathione have been insignificant.

### Disclosure of Invention

### Technical Problem

An aspect is to provide a cosmetic composition for skin condition improvement, including glutathione-containing exosome-like vesicles.

Another aspect is to provide a skin external preparation composition for skin condition improvement, including glutathione-containing exosome-like vesicles.

Another aspect is to provide a health functional food composition for skin condition improvement, including glutathione-containing exosome-like vesicles.

Another aspect is to provide a pharmaceutical composition for prevention or treatment of an inflammatory disease, including glutathione-containing exosome-like vesicles.

Another aspect is to provide use of glutathione-containing exosome-like vesicles for the manufacture of a cosmetic composition for skin condition improvement.

Another aspect is to provide use of glutathione-containing exosome-like vesicles for the manufacture of a skin external preparation composition for skin condition improvement.

Another aspect is to provide use of glutathione-containing exosome-like vesicles for the manufacture of a health functional food composition for skin condition improvement.

Another aspect is to provide use of glutathione-containing exosome-like vesicles for the manufacture of a medicament for prevention or treatment of an inflammatory disease.

Another aspect is to provide a method of improving, preventing, or treating a skin condition, including administering an effective amount of glutathione-containing exosome-like vesicles to a subject in need thereof.

### Solution to Problem

An aspect provides a cosmetic composition for skin condition improvement, including glutathione-containing exosome-like vesicles.

The term "glutathione" refers to a tripeptide including glutamate, cysteine, and glycine, and is generally reported as a bioactive substance having strong antioxidant activity.

The term "extracellular vesicle (EV)" refers to vesicles secreted by cells and released into the extracellular space, and may have a membrane structure that separates the intracellular space from the outside by a phospholipid bilayer having the same structure as a cell membrane and contains genetic materials and bioactive factors such as cytokines, growth factors, miRNA, DNA, and proteins therein. The EVs bind to other cells to deliver membrane components, mRNAs, miRNAs, and the like, and may accordingly act as extracellular carriers that mediate cell-to-cell communication by delivering these transmitting substances to receptor cells. In an exemplary embodiment, the EVs may include vesicles formed after encapsulating active ingredients inside cells when the cells burst due to turgor pressure, vesicles in which the membrane or the composition of active ingredients inside has been reconstituted as a result of bursting of multivesicular bodies under turgor pressure.

The term "exosome-like vesicles (ELVs)" may be interchangeably used with the term "exosome-like nanovesicles", and may refer to EVs having a size of tens to hundreds of nanometers. It is the broadest concept of vesicles, encompassing all nanoscale vesicles of which structures and compositions resemble those of exosomes. In an exemplary embodiment, the EVs may be ELVs.

In an embodiment, the ELVs may be ELVs derived from a plant, for example, ELVs derived from plant callus.

The term "plant" may be a whole plant or a part of a plant, and specifically, may include all of plant tissues, plant cells, and plant seeds.

In an embodiment, the plant may be any one part selected from the group consisting of flowers, leaves, stems, branches, fruits, fruit skins, and roots of a plant.

In an embodiment, the plant may be one or more selected from the group consisting of apples, grapes, desert roses, Centella asiatica, and edelweiss.

The term "callus" is a mass of unorganized parenchyma cells of plants, and refers to totipotent cells, including somatic cell embryos and plant stem cells. Also, it may be induced by transferring and culturing a part of the plant tissue in a tissue culture medium, and may be induced from any one site selected from the group consisting of flowers, leaves, stems, branches, fruits, fruit skins, and roots of a plant. The callus induction may be performed by methods known in the art. More specifically, the callus induction may be performed by culturing the plant tissue or a fragment thereof in a tissue culture medium. As the tissue culture medium, a variety of standard culture media for plants may be used, such as Murashige and Skoog (MS, 1962) media, SH media (Duchefa, Haarlem, Netherlands), N6 (Chu et al., 1975), B5 (Gamborg et al., 1968), NN (Nitsch and Nitsch, 1967) media, WHITE media, and the like. In addition, as the tissue culture medium, the one supplemented with a plant growth regulator may be used. As the plant growth regulator, materials known in the art may be used. For example, auxins such as 2,4-dichlorophenoxyacetic acid (2,4-D), 2,4,5-trichlorophenoxyacetic acid (2,4,5-T), 2-methoxy-3,6-dichlorobenzoic acid (dicamba), indol-3-acetic acid (IAA), indol-3-butyric acid (IBA), 2-methyl-4-chlorophenoxyacetic acid (MCPA), 1-naphthalylacetic acid (NAA), 2-naphthaleneacetic acid (NOA), 4-amino-2,5,6-trichloropicolinic acid (picloram), etc.; kinases such as 6-benzylaminopurine (BA), N6-(2-isopentyl)adenine (2iP), 6-furlaminopurine (Kinetin), 1-phenyl-3-(1,2,3-thiadiazol-5-yl)urea (Thidiazuron), 4-hydroxy-3-methyl-trans-2-butanepine (Zeatin), etc.; and derivatives thereof may be used. In addition, alkaloids, terpenoids, carotenoids, carbohydrates, phenol compounds, natural extracts, and the like, which act on differentiation and proliferation of cells, may be further added to the tissue culture medium.

In an embodiment, the plant callus may be cultured in an MS medium supplemented with NAA and BA.

In an embodiment, the plant callus may be any one part selected from the group consisting of flowers, leaves, stems, branches, fruits, fruit skins, and roots of a plant.

In an embodiment, the ELVs may be isolated from a lysate of the plant. Specifically, the ELVs may be isolated from a lysate of the plant callus. In an embodiment, the lysate of the plant callus may be a lysate of a mixed culture medium of the plant callus. In another embodiment, the lysate of the plant callus may be a lysate of the callus recovered after removing the culture supernatant from the mixed culture medium of the plant callus. In another embodiment, the lysate of the plant callus may be a lysate of the culture supernatant recovered after removing the callus from the mixed culture medium of the plant callus. To isolate the callus or the culture supernatant from the mixed culture medium of the plant callus, a standard sieve and a mesh net may be used. Here, those having a pore size of 100 µm to 200 µm may be appropriate, but the pore size is not limited thereto. Conventional methods used in the art, such as using a centrifuge, a filtration device, a qualitative filter paper, and the like, may be used to isolate the callus and culture supernatant.

The term "mixed culture medium of callus" may refer to a whole medium containing the callus, metabolites thereof, and a surplus nutrients, obtained by culturing the callus for a certain period of time in a medium capable of supplying nutrients such that the callus can grow and survive in vitro. For example, the callus may be obtained by culturing in a medium at any temperature of higher than 10 °C or lower than 40 °C for a certain period of time, for example, 1 to 40 days. The culture medium and culture conditions for culturing the callus may be appropriately selected or modified by those skilled in the art.

The term "culture supernatant" may refer to only a liquid in the upper layer except for a portion that has settled in the lower layer by allowing the culture medium to remain still for a certain period of time, or may refer to only a liquid in the upper layer except for precipitation in the lower portion by centrifuging the culture medium.

The term "lysate" may refer to a product obtained by lysis using physical force. In an embodiment, the lysate may be produced by applying pressure (turgor pressure), and may be, for example, obtained by using a high-pressure homogenizer. When glutathione is added in the process of physically obtaining a lysate of the callus by applying pressure to the plant callus with a high-pressure homogenizer, the glutathione may be induced to be encapsulated in ELVs during the lysis process, and thus the ELVs isolated from the lysate of the plant callus may be usefully utilized as a carrier that contains glutathione therein.

The term "containing glutathione" may be used interchangeably with the terms "encapsulating glutathione", "glutathione-loaded", or "glutathione-entrapped".

In an embodiment, the skin condition improvement may be anti-inflammation.

The term "anti-inflammation" may be used interchangeably with "suppression or alleviation of inflammation", and may refer to all actions that alleviate inflammatory conditions.

The term "improvement" may refer to all actions that at least reduce a parameter related to alleviation or treatment of a condition, for example, the severity of symptoms.

In an embodiment, the ELVs may be isolated by ultrafiltration using a 20 kDa to 500 kDa molecular weight cutoff (MWCO) membrane. Specifically, the ELVs may be isolated by ultrafiltration from a high-pressure homogenized lysate of the plant callus. More specifically, the ELVs may be isolated by ultrafiltration from a sample obtained by centrifuging a high-pressure homogenized lysate of the mixed culture medium of the plant callus to remove cell-related residues and filtering the obtained supernatant through a 0.45 µm filter.

In an embodiment, the ELVs may have a diameter of 20 to 400nm. For example, the diameter may be 20 nm to 350 nm, 20 nm to 300 nm, 20 nm to 250 nm, 20 nm to 200 nm, 50 nm to 400 nm, 50 nm to 350 nm, 50 nm to 300 nm, 50 nm to 250 nm, 50 nm to 200 nm, 80 nm to 400 nm, 80 nm to 350 nm, 80 nm to 300 nm, 80 nm to 250 nm, 80 nm to 200 nm, 80 nm to 150 nm, 80 nm to 130 nm, 80 nm to 120 nm, 80 nm to 100 nm, 100 nm to 400 nm, 100 nm to 350 nm, 100 nm to 300 nm, 100 nm to 200 nm, 10 nm to 20 nm, It may be 150 nm, 100 nm to 130 nm, 100 nm to 120 nm, 150 nm to 400 nm, 150 nm to 350 nm, 150 nm to 300 nm, 150 nm to 250 nm, 150 nm to 200 nm, 200 nm to 400 nm, 200 nm to 350 nm, 200 nm to 300 nm, 200 nm to 250 nm, 250 nm to 400 nm, 250 nm to 350 nm, 250 nm to 300 nm, 300 nm to 400 nm, or 300 nm to 350 nm.

In an embodiment, the glutathione may be contained in an amount of 0.00001 to 50 wt%, for example, 0.00001 to 40 wt%, 0.00001 to 30 wt %, or 0.00001 to 20 wt%, based on the total weight of the ELVs. When the amount of the glutathione is less than the above amount, a significant effect may not appear, and when the amount is greater than the above amount, exosome stability issues may be caused.

The composition may include the glutathione-containing ELVs in an amount of about 0.000000001 wt% to 80 wt%, for example, 0.000000001 wt% to 60 wt%, 0.000000001 wt% to 40 wt%, 0.000000001 wt% to 30 wt%, 0.000000001 wt% to 20 wt%, 0.000000001 wt% to 10 wt%, 0.000000001 wt% to 5 wt%, 0.000000001 wt% to 0.001 wt%, 0.000000001 wt% to 0.0001 wt%, 0.000000001 wt% to 0.00001 wt%, 0.0001 wt% to 80 wt%, 0.0001 wt% to 60 wt%, 0.0001 wt% to 40 wt%, 0.0001 wt% to 30 wt%, 0.0001 wt% to 20 wt%, 0.0001 wt% to 10 wt%, 0.0001 wt% to 5 wt%, 0.001 wt% to 80 wt%, 0.001 wt% to 60 wt%, 0.001 wt% to 40 wt%, 0.001 wt% to 30 wt%, 0.001 wt% to 20 wt%, 0.001 wt% to 10 wt%, 0.001 wt% to 5 wt%, 0.01 wt% to 80 wt%, 0.01 wt% to 60 wt%, 0.01 wt% to 40 wt%, 0.01 wt% to 30 wt%, 0.01 wt% to 20 wt%, 0.01 wt% to 10 wt%, 0.01 wt% to 5 wt%, 0.1 wt% to 80 wt%, 0.1 wt% to 60 wt%, 0.1 wt% to 40 wt%, 0.1 wt% to 30 wt%, 0.1 wt% to 20 wt%, 0.1 wt% to 10 wt%, 0.1 wt% to 5 wt%%, based on the total weight of the composition.

In an embodiment, the composition may contain the glutathione-containing ELVs in a concentration of 10³ vesicles/ml to 10¹² vesicles/ml, for example, 10³ vesicles/ml to 10¹¹ vesicles/ml, 10³ vesicles/ml to 10¹⁰ vesicles/ml, 10³ vesicles/ml to 10⁹ vesicles/ml, 10³ vesicles/ml to 10⁸ vesicles/ml, 10³ vesicles/ml to 10⁷ vesicles/ml, 10³ vesicles/ml to 10⁶ vesicles/ml, 10³ vesicles/ml to 10⁵ vesicles/ml, 10³ vesicles/ml to 10⁴ vesicles/ml, 10⁴ vesicles/ml to 10¹¹ vesicles/ml, 10⁴ vesicles/ml to 10¹⁰ vesicles/ml, 10⁴ vesicles/ml to 10⁹ vesicles/ml, 10⁴ vesicles/ml to 10⁸ vesicles/ml, 10⁴ vesicles/ml to 10⁷ vesicles/ml, 10⁴ vesicles/ml to 10⁶ vesicles/ml, 10⁴ vesicles/ml to 10⁵ vesicles/ml, 10⁵ vesicles/ml to 10¹¹ vesicles/ml, 10⁵ vesicles/ml to 10¹⁰ vesicles/ml, 10⁵ vesicles/ml to 10⁹ vesicles/ml, 10⁵ vesicles/ml to 10⁸ vesicles/ml, 10⁵ vesicles/ml to 10⁷ vesicles/ml, 10⁵ vesicles/ml to 10⁶ vesicles/ml, 10⁶ vesicles/ml to 10¹² vesicles/ml, 10⁶ vesicles/ml to 10¹¹ vesicles/ml, 10⁶ vesicles/ml to 10¹⁰ vesicles/ml, 10⁶ vesicles/ml to 10⁹ vesicles/ml, 10⁶ vesicles/ml to 10⁸ vesicles/ml, 10⁶ vesicles/ml to 10⁷ vesicles/ml, 10⁷ vesicles/ml to 10¹² vesicles/ml, 10⁷ vesicles/ml to 10¹¹ vesicles/ml, 10⁷ vesicles/ml to 10¹⁰ vesicles/ml, 10⁷ vesicles/ml to 10⁹ vesicles/ml, 10⁷ vesicles/ml to 10⁸ vesicles/ml, 10⁸ vesicles/ml to 10¹² vesicles/ml, 10⁸ vesicles/ml to 10¹¹ vesicles/ml, 10⁸ vesicles/ml to 10¹⁰ vesicles/ml, 10⁸ vesicles/ml to 10⁹ vesicles/ml, 10⁹ vesicles/ml to 10¹² vesicles/ml, 10⁹ vesicles/ml to 10¹¹ vesicles/ml, 10⁹ vesicles/ml to 10¹⁰ vesicles/ml, 10¹⁰ vesicles/ml to 10¹² vesicles/ml, 10¹⁰ vesicles/ml to 10¹¹ vesicles/ml, or 10¹¹ vesicles/ml to 10¹² vesicles/ml. When the glutathione-containing ELVs are contained in the composition in the amount less than the above range, a significant effect of improving skin conditions may not appear, and when included in the amount greater than the above range, formulation stability may be deteriorated.

The cosmetic composition may be prepared in a formulation including a lotion (skin lotion), a skin softener, a skin toner, an astringent, a lotion, a milk lotion, a moisturizing lotion, a nourishing lotion, a massage cream, a nourishing cream, a moisturizing cream, an eye cream, a hand cream, a foundation, an essence, a nourishing essence, an eye essence, a pack, a soap, a cleansing foam, a cleansing lotion, a cleansing cream, a body lotion, a body cream, a body cleanser, a suspension, a gel, a powder, a paste, a mask pack, a sheet, or an aerosol composition. The composition of such a formulation may be prepared according to a conventional method in the art.

The components included in the cosmetic composition may include components commonly used in the cosmetic composition in addition to the composition as the active ingredient, and may include, for example, conventional adjuvants and carriers such as a stabilizer, a solubilizer, a vitamin, a pigment, and a fragrance.

Another aspect provides a skin external preparation composition for skin condition improvement, including glutathione-containing ELVs. In the skin external preparation composition, the glutathione, the ELVs, the skin condition improvement, and the composition are as described above.

In an embodiment, the ELVs may be ELVs derived from a plant, for example, ELVs derived from plant callus. The plant, callus, and the like are as described above.

The skin external preparation may be a cream, a gel, an ointment, a skin emulsifier, a skin suspension, a transdermal patch, a drug-containing bandage, a lotion, or a combination thereof. The skin external preparation may be appropriately mixed with components used in skin external preparations, such as cosmetics or pharmaceuticals, for example, aqueous components, oily components, powder components, alcohols, moisturizers, thickeners, ultraviolet absorbers, whitening agents, preservatives, antioxidants, surfactants, fragrances, colorants, various skin nutrients, or a combination thereof, if necessary. The skin external preparation may be appropriately mixed with a metal sequestering agent, such as disodium edetate, trisodium edetate, sodium citrate, sodium polyphosphate, sodium metaphosphate, gluconic acid, etc., a drug such, as caffeine, tannin, belapamyl, licorice extract, glyblidine, fruit hot water extract of calin, various herbals, tocopherol acetate, glytilitic acid, tranexamic acid, and derivatives thereof or salts thereof, and saccharides, such as vitamin C, magnesium ascorbate phosphate, glucoside ascorbate, albutin, kojic acid, glucose, fructose, trehalose, etc.

Another aspect provides a health functional food for skin condition improvement, including glutathione-containing ELVs. In the health functional food, the glutathione, the ELVs, the skin condition improvement, and the composition are as described above.

In an embodiment, the ELVs may be ELVs derived from a plant, for example, ELVs derived from plant callus. The plant, callus, and the like are as described above.

The health functional food composition may be used with only ELVs, or in combination with other foods or food ingredients, and may be appropriately used according to a conventional method. A mixing amount of the active ingredient may be appropriately determined according to the purpose of use (prevention, health, or therapeutic treatment). In general, when food or beverages are prepared, the composition of the present specification may be added in an amount of 15 parts by weight or less based on the raw material. Types of the health functional food are not particularly limited. Among the types of health functional foods, a beverage composition may contain, as additive components, various flavoring agents or natural carbohydrates as in typical beverages. The natural carbohydrates may be monosaccharides such as glucose and fructose, disaccharides such as maltose and sucrose, polysaccharides such as dextrin and cyclodextrin, and sugar alcohols such as xylitol, sorbitol, and erythritol. As the sweetening agent, natural sweetening agents such as thaumatin and stevia extract, and synthetic sweetening agents such as saccharin and aspartame may be used. The health food composition may also contain nutrients, vitamins, electrolytes, flavoring agents, colorants, pectic acid and salts thereof, alginic acid and salts thereof, organic acids, protective colloid thickeners, pH adjusters, stabilizers, preservatives, glycerin, alcohols, carbonating agents used in carbonated beverages, or a combination thereof. The health functional food composition may also contain natural fruit juice, fruit juice beverages, fruit flesh for the production of vegetable beverages, or a combination thereof.

Another aspect provides a pharmaceutical composition for prevention or treatment of an inflammatory disease, the pharmaceutical composition including glutathione-containing ELVs. In the pharmaceutical composition, the glutathione, the ELVs, wounds, and configurations are as described above.

In an embodiment, the ELVs may be ELVs derived from a plant, for example, ELVs derived from plant callus. The plant, callus, and the like are as described above.

The "inflammatory disease" may be an inflammatory skin disease, an allergic inflammatory disease, an inflammatory eye disease, an inflammatory osteoarthritis disease, an inflammatory muscle disease, or an inflammatory bowel disease, and the "inflammatory skin disease" may be any one selected from the group consisting of atopic dermatitis, psoriasis, contact dermatitis, eczematous dermatitis, photodermatitis, seborrheic dermatitis, dermatitis herpetiformis, lichen planus, lichen sclerosus, pyoderma gangrenosum, pemphigus, epidermolysis bullosa, allergy, and hypersensitivity.

The term "prevention" may comprehensively refer to prevention of a disease in advance or reduction of the possibility of occurrence or frequency of occurrence, by administering the composition in a pharmaceutically effective amount. For example, it may involve reducing the likelihood of occurrence of a disease or reducing the likelihood of recurrence, in a patient at risk of developing a disease or a patient who has already developed a disease. The "pharmaceutically effective amount" may be easily determined by those skilled in the art according to factors well known in the medical field, such as a disease type, age, weight, health, and gender of a patient, drug sensitivity of a patient, a route of administration, a method of administration, a frequency of administration, duration of treatment, combination, or concomitant drugs.

The term "treatment" may comprehensively refer to amelioration of a disease by administering a pharmaceutically effective amount of the composition. It may refer to relief or healing of symptoms of the disease for a shortened time compared to natural healing, and may refer to amelioration of a single symptom or most symptoms caused by the disease. The pharmaceutically effective amount is as described above.

The pharmaceutical composition may further include a pharmaceutically acceptable diluent or carrier. The diluent may include lactose, corn starch, soybean oil, microcrystalline cellulose, or mannitol, and the lubricant may include magnesium stearate, talc, or a combination thereof. The carrier may include an excipient, a disintegrant, a binder, a lubricant, or a combination thereof. The excipient may include microcrystalline cellulose, lactose, low-substituted hydroxycellulose, or a combination thereof. The disintegrant may include carboxymethylcellulose calcium, sodium starch glycolate, anhydrous monohydrogen phosphate, or a combination thereof. The binder may include polyvinylpyrrolidone, low-substituted hydroxypropyl cellulose, hydroxypropyl cellulose, or a combination thereof. The lubricant may include magnesium stearate, silicon dioxide, talc, or a combination thereof.

Another aspect is to provide use of the glutathione-containing exosome-like vesicles for the manufacture of a cosmetic composition for skin condition improvement. Regarding the use, overlapping contents such as the glutathione-containing exosome-like vesicles, the skin condition improvement, and the cosmetic compositions are as described above.

Another aspect is to provide use of the glutathione-containing exosome-like vesicles for the manufacture of a skin external preparation composition for skin condition improvement. Regarding the use, overlapping contents such as the glutathione-containing exosome-like vesicles, the skin condition improvement, and the skin external preparations are as described above.

Another aspect is to provide use of the glutathione-containing exosome-like vesicles for the manufacture of a health functional food composition for skin condition improvement. Regarding the use, overlapping contents such as the glutathione-containing exosome-like vesicles, the skin condition improvement, and the skin external preparation composition are as described above.

Another aspect is to provide use of the glutathione-containing exosome-like vesicles for the manufacture of a medicament for prevention or treatment of an inflammatory disease. Regarding the use, overlapping contents such as the glutathione-containing exosome-like vesicles, the inflammatory disease, the prevention and treatment, and the pharmaceutical, and the like are as described above.

Another aspect is to provide a method of improving, preventing, or treating a skin condition, the method including administering an effective amount of glutathione-containing exosome-like vesicles to a subject in need thereof. Regarding the method, overlapping contents such as the glutathione-containing exosome-like vesicles, the skin condition improvement, and the prevention and treatment, and the like are as described above.

The terms "administering", "introducing", and "implanting" are used interchangeably, and may refer to arrangement of the composition according to an embodiment in a subject by a method or pathway that results in at least partial localization of the composition to a desired site.

The administration may be performed by a method known in the art. The administration may be directly administered to the subject by any means, for example, intravenous, intramuscular, oral, transdermal, mucosal, intranasal, intratracheal, or subcutaneous routes. The administration may be administered systemically or locally.

The subject may be a mammal, for example, a human, a cow, a horse, a pig, a dog, a sheep, a goat, or a cat. The subject may be the one in need of the skin condition improvement, for example, suppression of skin inflammation.

The term "effective amount" may refer to an amount effective enough to exhibit the aforementioned effects.

The administration may be performed by administering the composition according to an embodiment per subject at a dosage of 0.1 mg to 1,000 mg, for example, 0.1 mg to 500 mg, 0.1 mg to 100 mg, 0.1 mg to 50 mg, 0.1 mg to 25 mg, 1 mg to 1,000 mg, 1 mg to 500 mg, 1 mg to 100 mg, 1 mg to 50 mg, 1 mg to 25 mg, 5 mg to 1,000 mg, 5 mg to 500 mg, 5 mg to 100 mg, 5 mg to 50 mg, 5 mg to 25 mg, 10 mg to 1,000 mg, 10 mg to 500 mg, 10 mg to 100 mg, 10 mg to 50 mg, or 10 mg to 25 mg. However, the dosage may be prescribed in various ways by factors such as a formulation method, an administration method, age, body weight, gender, and pathological conditions of a patient, food, an administration time, an administration route, an excretion rate, and a response sensitivity, and those skilled in the art may appropriately adjust the dosage in consideration of these factors. The frequency of administration may be once a day or at least twice a day within the range of clinically acceptable side effects, and the administration may be performed at a single site or at least two sites, daily or at intervals of 2 to 5 days. The total number of administration days may be from 1 to 30 days per treatment. If necessary, the same treatment may be repeated after a suitable period of time. For animals other than humans, the same dosage per kg as for humans may be used, or for example, a dosage converted from the aforementioned dosage by the volume ratio (e.g., an average value) of the organ (heart, etc.) between a target animal and a human may be used.

### Advantageous Effects of Invention

The glutathione-containing exosome-like vesicles according to an aspect may effectively deliver glutathione into cells, exhibiting an excellent anti-inflammatory effect, and thus may be usefully utilized as a composition for skin condition improvement.

### Brief Description of Drawings

FIG. 1 shows results of confirming expression levels of IL-1β mRNA after treating keratinocytes with glutathione-containing exosome-like vesicles derived from plant callus (Example) or with glutathione (Comparative Example 1), each at a concentration of 1.0 %.
FIG. 2 shows results of confirming expression levels of TSLP mRNA after treating keratinocytes with glutathione-containing exosome-like vesicles derived from plant callus (Example) or with glutathione (Comparative Example 1), each at a concentration of 1.0 %.

### Mode for the Invention

Hereinafter, the present disclosure will be described in more detail through Examples. However, these Examples are provided to illustrate the present disclosure, and the scope of the present invention is not limited to these Examples.

### Example. Preparation of glutathione-containing exosome-like vesicles derived from plant callus

### (1) Induction of apple callus

The apple fruits (*Malus domestica*) used in the experiment were obtained from the Neunggeum Agricultural Cooperative. Apple fruits were washed in running water, and then subjected to first sterilization in 70 % ethanol for 1 minute. Following second sterilization in 1 % sodium hypochlorite for 10 minutes, the apple fruits were washed three times with sterile distilled water. The sterilized fruit tissue was cut into a size of 0.5 cm², and then cultured in an MS medium supplemented with growth regulators including 4.5 mg/L of 1-naphthalylacetic acid (NAA) and 2.5 mg/L of 6-benzylaminopurine (BA). The culturing was performed in the dark while adjusting the pH of the medium to 5.8 and adjusting the external temperature to 27±3 °C. Afterwards, 4 g of apple callus was inoculated into an Erlenmeyer flask containing 50 mL of a liquid medium of the same medium composition, and maintained in the dark, and then cultured on a stirrer (J-MBB2, JISICO, Republic of Korea) at a speed of 90 rpm. The culturing was performed for a total of 3 to 4 weeks.

### (2) Preparation of glutathione-containing exosome-like vesicles derived apple callus

An isotonic solution was added to the mixed culture medium of apple callus cultured in the liquid medium of Example (1), and then lysed using a homogenizer (Homogenizer, T50D, IKA, Aachen, Germany). After adding glutathione to the obtained lysate, the process of applying a pressure of 1000 bar using a high-pressure homogenizer (Microfludizer, MN600P-300, Picomax, Korea) was repeated three times such that the cell walls and the cell membranes were lysed due to the turgor pressure, causing exosome-like vesicles inside the plant cells to be released. At the same time, the released exosome-like vesicles burst by the turgor pressure, collecting glutathione and inducing formation of glutathione-encapsulated vesicles. The lysate of the mixed culture medium thus obtained was centrifuged at 4 °C at 10,000 Xg for 30 minutes to remove cell wall debris and obtain the supernatant. The obtained supernatant was filtered through a 0.45 µm sterile filter to further remove any remaining cell debris. The filtered supernatant was subjected to ultrafiltration (Pellicon^{®} 2 and 3 Mini Holder, Merck, Germany) through a 100 kDa MWCO membrane to isolate and concentrate glutathione-containing exosome-like vesicles of at least 100 kDa. To additionally remove impurities, the vesicles were sterile filtered through a 0.2 µm sterile filter. The finally isolated glutathione-containing exosome-like vesicles were diluted 200 times and used in the following experiments.

### Comparative Example 1. Preparation of aqueous glutathione solution

Glutathione having the same amount as in Example above was dissolved in water to prepare an aqueous solution.

### Comparative Example 2. Preparation of glutathione-containing exosome-like vesicles derived from human stem cells

Glutathione-encapsulated exosome-like vesicles derived from human stem cells were prepared in the same manner as in the Example above, except that exosome-like vesicles derived from human stem cells were used instead of exosome-like vesicles derived from the plant callus of the Example above.

Specifically, human adipose-derived stem cells were cultured under conditions of 5 % CO₂ and 37 °C according to a cell culture method known in the art to which the present invention belongs. Then, the cells were washed with phosphate-buffered saline (purchased from ThermoFisher Scientific), and cultured in a medium without serum and phenol red. After adding the same amount of glutathione as in Example to the human adipose-derived stem cell culture medium cultured for 72 hours, the process of applying a pressure of 1000 bar by using a high-pressure homogenizer (Microfludizer, MN600P-300, Picomax, Korea) was repeated three times to induce the formation of glutathione-encapsulated vesicles. By ultrafiltration (Pellicon^{®} 2 and 3 Mini Holder, Merck, Germany) through a 100 kDa MWCO membrane, glutathione-containing exosome-like vesicles of at least 100 kDa were isolated and concentrated. To additionally remove impurities, the vesicles were sterile filtered through a 0.2 µm sterile filter. The finally isolated glutathione-containing exosome-like vesicles were diluted 200 times and used in the following experiments.

### Experimental Example 1. Evaluation of anti-inflammatory effects

To confirm the anti-inflammatory effects of the glutathione-containing exosome-like vesicles derived from the plant callus prepared in the Example above, the expression levels of inflammatory cytokines, i.e., interleukin-1β (IL-1β) and thymic stromal lymphopoietin (TSLP), were measured at the mRNA level after treating keratinocytes with the glutathione-containing exosome-like vesicles derived from the plant callus of the Example above.

### 1-1. Human keratinocyte culture

HaCaT cells, which are human keratinocytes, were cultured in a 100 mm dish containing a Dulbecco's modified Eagle's medium (DMEM) medium supplemented with 10 % fetal bovine serum (FBS) and 1 % penicillin. The samples were prepared by filtering through a 0.2 µm-syringe filter, and each sample was added to the cell culture medium so as to have a final concentration of 1% (v/v). As a positive control group, 1 µM dexamethasone having an anti-inflammatory action was used. The culture medium containing the sample was cultured for 24 hours in an incubator maintained under conditions of a temperature of 37 °C, 5 % CO₂ concentration, and 95 % humidity, and then treated with 10 µg/ml of poly I:C and 10 ng/ml of IL-4, which are immunostimulants, and cultured for 4 hours.

### 1-2. Analysis of expression of inflammation-inducing cytokines

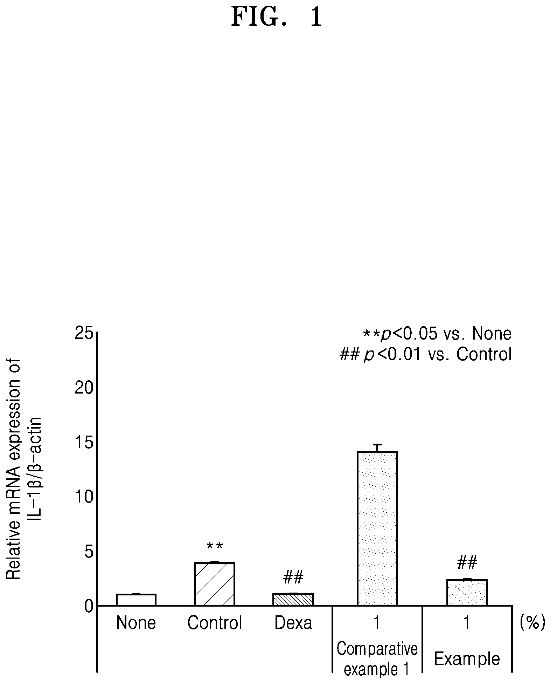

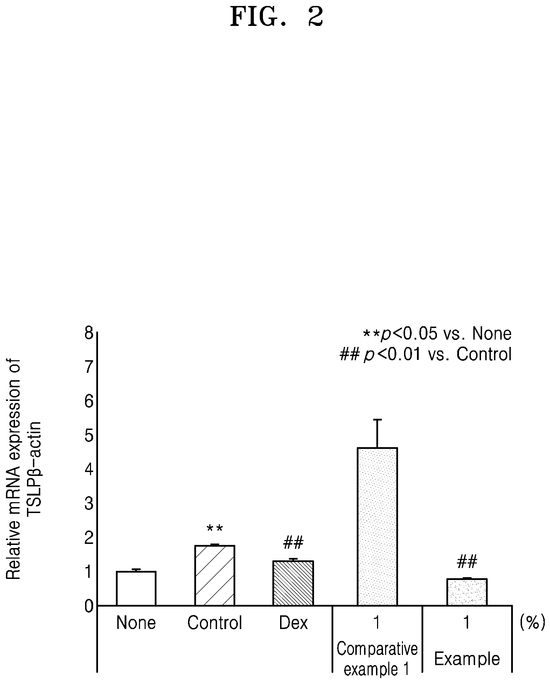

The cells cultured in Experimental Example 1-1 were recovered, washed with a cooled phosphate buffer solution (PBS), and total RNA was extracted by using a PureLink^{®} RNA Mini Kit (Thermo Fisher Scientific). Specific RNA extraction was performed according to the manual provided with the product. Changes in the gene expression were measured by quantitative real-time PCR (qRT-PCR), a method that involves labeling a fluorescent substance to DNA products amplified by polymerase chain reaction (PCR) and continuously detecting the fluorescent substance. To quantify the expression of inflammatory response factors, i.e., IL-1β and TSLP genes, the fluorescence values emitted by PCR products were measured through SYBR green (Thermo Fisher Scientific). A reaction solution was prepared by mixing in a PCR tube 0.2 µM primer, 50 mM KCl, 20 mM Tris/HCl pH 8.4, 0.8 mM dNTP, 0.5U Extaq DNA polymerase, 3 mM MgCl2, and 1×SYBR green. After preliminary denaturation at 94 °C for 3 minutes by using a PCR machine, denaturation, annealing, and polymerization were performed at 94 °C for 30 seconds, 58 °C for 30 seconds, and 72 °C for 30 seconds, respectively, for a total of 40 cycles. After each cycle was completed, the fluorescence intensity was measured. The fluorescence intensities of the IL-1β and TSLP genes were normalized to actin values, with the control group set as 100 %, to compare the differences and then measure the expression level of each gene. The results are shown in FIGS. 1 and 2. Primer sequences used in the experiment are shown in Table 1 below.

**[Table 1]**

| Gene | Forward primer | Reverse primer |
|---|---|---|
| *IL-1β* | 5'-GTCATTCGCTCCCACATTCT-3' (SEQ ID NO: 1) | 5'-ACTTCTTGCCCCCTTTGAAT-3' (SEQ ID NO: 2) |
| *TSLP* | 5'- ATGTTCGCCATGAAAACTAAGGC- 3' (SEQ ID NO: 3) | 5'-GCGACGCCACAATCCTTGTA - 3' (SEQ ID NO: 4) |
| *Actin* | 5'-GGCCATCTCTTGCTCGAAGT-3' (SEQ ID NO: 5) | 5'-GACACCTTCAACACCCCAGC-3' (SEQ ID NO: 6) |

FIG. 1 shows the results of confirming the expression levels of IL-1β mRNA after treating keratinocytes with the glutathione-containing exosome-like vesicles derived from the plant callus (Example) or with glutathione (Comparative Example 1), each at a concentration of 1.0 %. FIG. 2 shows the results of confirming the expression levels of TSLP mRNA after treating keratinocytes with the glutathione-containing exosome-like vesicles derived from the plant callus (Example) or with glutathione (Comparative Example 1), each at a concentration of 1.0 %. As a result, as shown in FIGS. 1 and 2, it was confirmed that the expression of inflammation-related factors was significantly reduced in a case of treatment with glutathione loaded into the plant-derived exosome-like vesicles compared to a case of treatment with glutathione alone at the same concentration (Comparative Example 1). That is, in a case of treatment with glutathione by encapsulation in the plant callus-derived exosome-like vesicles, glutathione which is the active ingredient can be effectively delivered into cells, compared to a case of treatment with glutathione alone, indicating effective suppression of inflammation.

### Experimental Example 2. Evaluation of carrier-dependent stability

To evaluate the carrier-dependent stability of glutathione, the glutathione-containing exosome-like vesicles derived from the plant callus of Example, the glutathione-containing exosome-like vesicles derived from the human stem cells of Comparative Example 2, and the glutathione aqueous solution of Comparative Example 1 were stored for 1 year under storage conditions in which light was blocked at 25 °C, and then the concentration of the encapsulated glutathione was measured by high performance liquid chromatography (HPLC) analysis. At this time, reduced glutathione was used as a standard. Glutathione is a strong antioxidant that removes active oxygen as being oxidized upon contact with active oxygen, thereby causing glutathione to be unstable. Loading glutathione in a structure such as exosome-like vesicles may improve stability by reducing the chance of exposure to active oxygen. Therefore, the stability of glutathione loaded in the exosome-like vesicle was confirmed through the degree of oxidation of reduced glutathione.

Specifically, a reduced glutathione (GSH) standard (500 ppm) was dissolved in a 50 ml conical tube with tertiary distilled water, and then prepared as being diluted at 500 ppm, 250 ppm, 125 ppm, 62.5 ppm, and 31.25 ppm. Each of the diluted standard, the sample of Comparative Example 1 diluted in tertiary distilled water, the sample of Example diluted in tertiary distilled water, and the sample of Comparative Example 2 diluted in tertiary distilled water was filtered through a 0.2 µm-syringe filter, and the filtrate was put into a vial for HPLC. 10 ul was injected into a C18 column, and the samples were eluted at a flow rate of 1 ml/min, and the values were measured by using a DAD detector. The results are shown in Table 2 below.

**[Table 2]**

| Relative concentration of reduced glutathione (GSH) | Immediately after loading | 6 months after loading | 1 year after loading |
|---|---|---|---|
| Comparative Example 1 | 1.00 | 0.21 | 0.06 |
| Comparative Example 2 | 1.00 | 0.81 | 0.73 |
| Example | 1.00 | 0.92 | 0.85 |

As a result, as shown in Table 2, in the case of the glutathione not loaded on the exosome-like vesicles (Comparative Example 1), the glutathione concentration was significantly reduced to about 20 % compared to the initial concentration in 6 months, and reduced to about 6 % after 1 year, but the glutathione concentration was maintained at about 70 % or more in the case of the glutathione loaded on the exosome-like vesicles (Example and Comparative Example 2). In particular, when the plant callus-derived exosome-like vesicles were used as a carrier for glutathione (Example), a significantly high concentration of about 85% or more was achieved, confirming that glutathione was stably maintained for a long period of time. That is, the plant callus-derived exosome-like vesicles according to an aspect are meant to be an effective carrier capable of stably mataining glutathione therein for a long period of time.

## Claims

1. A cosmetic composition for skin condition improvement, comprising glutathione-containing exosome-like vesicles.

2. The cosmetic composition of claim 1, wherein the exosome-like vesicles are exosome-like vesicles derived from a plant.

3. The cosmetic composition of claim 2, wherein the plant is any one part selected from the group consisting of flowers, leaves, stems, branches, fruits, fruit skins, and roots of a plant.

4. The cosmetic composition of claim 1, wherein the skin condition improvement is anti-inflammation.

5. The cosmetic composition of claim 1, wherein the plant-derived exosome-like vesicles are isolated from a plant lysate.

6. The cosmetic composition of claim 1, wherein the exosome-like vesicles are isolated by ultrafiltration using a 20 kDa to 500 kDa molecular weight cutoff (MWCO) membrane.

7. A skin external preparation composition for skin condition improvement, comprising glutathione-containing exosome-like vesicles.

8. A health functional food composition for skin condition improvement, comprising glutathione-containing exosome-like vesicles.

9. A pharmaceutical composition for prevention or treatment of an inflammatory disease, comprising glutathione-containing exosome-like vesicles.
